# EUROPEAN PATENT SPECIFICATION

(11) **EP 2 654 729 B1**
(45) Date of publication and mention of the grant of the patent: **04.05.2016**
(21) Application number: 11805851.0
(22) Date of filing: 22.12.2011
(51) Int. Cl.: A61K 9/20, A61K 9/28, A61K 31/401, A61K 31/4422

(54) **HOMOGENOUS PHARMACEUTICAL ORAL DOSAGE FORMS COMPRISING LERCANIDIPINE AND ENALAPRIL OR THEIR PHARMACEUTICALLY ACCEPTABLE SALTS TOGETHER WITH AN ORGANIC ACID**
ORALE HOMOGENE PHARMAZEUTISCHE ZUBEREITUNG ENTHALTEND LERCANIDIPINE UND ENALAPRIL ODER IHRE PHARMAZEUTISCHE SALZE ZUSAMMEN MIT EINER ORGANISCHEN SÄURE
FORMES PHARMACEUTIQUES ORALES HOMOGÈNES COMPRENANT DE LA LERCANIDIPINE ET DE L'ÉNALAPRIL OU LEURS SELS PHARMACEUTIQUEMENT ACCEPTABLES AINSI QU'UN ACIDE ORGANIQUE

(30) Priority: 24.12.2010 SI 201000451; 21.04.2011 SI 201100142
(43) Date of publication of application: 30.10.2013
(73) Proprietor: KRKA, d.d., Novo mesto, 8501 Novo mesto (SI)
(72) Inventor: SEDMAK, Gregor, 1000 Ljubljana (SI); KROSELJ, Vesna, 8000 Novo mesto (SI); SKUBE, Maja Kincl, 8000 Novo mesto (SI); JURSIC, Urska, 8322 Stopice (SI)
(74) Representative: Uexküll & Stolberg
(86) International application number: PCT/EP2011/073884
(87) International publication number: WO 2012/085249

(56) References cited:
- WO-A1-01/19348
- WO-A2-00/66116
- WO-A2-2005/053689
- CN-A- 101 836 982
- US-A- 4 830 853

## Description

### Field of the Invention

The invention relates to homogenous pharmaceutical oral dosage forms of the active substances lercanidipine and enalapril, as well the pharmacologically acceptable salts thereof, characterized in that the pharmaceutical oral dosage forms comprise an organic acid to stabilize and improve the dissolution profiles of both enalapril and lercanidipine, and their pharmaceutically acceptable salts.

### Background of the Invention

Lercanidipine (methyl 1,1,N-trimethyl-N-(3,3-diphenylpropyl)-2-aminoethyl 1,4-dihydro-2,6-dimethyl-4-(3-nitrophenyl)pyridine-3,5-dicarboxylate) is a highly lipophilic dihydropyridine calcium antagonist with a long duration of action and high vascular selectivity. It has been approved for the treatment of hypertension and has been marketed in several European countries under the trademark Zanidip® since 1996. The molecular formula of lercanidipine is set forth in Formula (I) below.

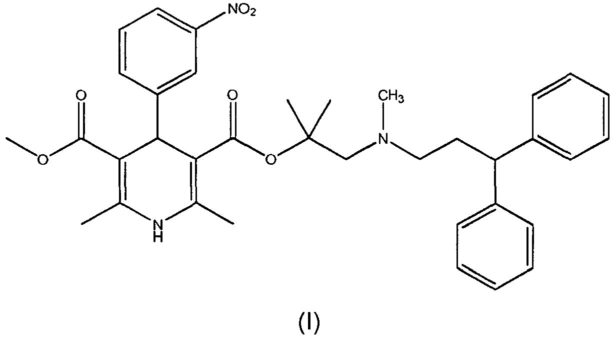

Lercanidipine has been studied in the dosage ranging from 2 to 80 mg. It is normally administered in a dosage of about 10 mg to about 20 mg once or twice daily, the recommended maximum dose being about 30 mg once or twice daily. The preparation of lercanidipine adsorbates is described by WO 2008/068777.

The hydrochloride salt of lercanidipine is commercially available. Methods of making both lercanidipine free base and its hydrochloride salt have been described previously along with methods of resolving lercanidipine into individual enantiomers in U.S. Patent Nos. 4,705,797; 5,767,136; 4,968,832; 5,912,351; and 5,696,139.

Enalapril is an ACE inhibitor useful in treating hypertension and heart failure. It is disclosed in U.S. Pat. No. 4,374,829, U.S. Pat. Nos. 4,374,829, 4,472,380 and 4,510,083 as well as methods for its preparation. In U.S. 5,350,582 a stable formulation of an enalapril salt is disclosed, as well as in U.S. 5,573,780, U.S. 5,690,962, U.S. 6,555,551, EP 425 892 and U.S. 6,555,551.

The molecular formula of enalapril is set forth in Formula (II) below.

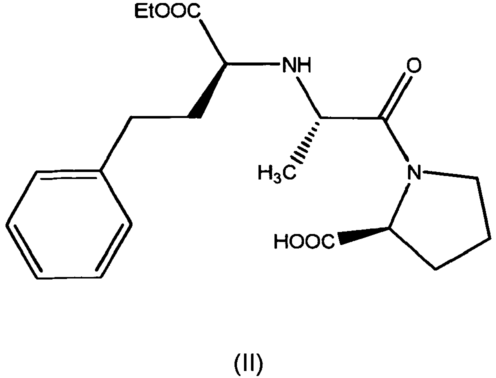

As it is known, many compounds that inhibit ACE (Angiotensin-Converting Enzyme) have poor stability either in form of free acids or salts if they are in a pharmaceutical dosage form. These compounds easily decompose first of all by hydrolysis and intramolecular cyclization, but the amount of other decomposition products not identified in many cases may be also significant. This is particularly true in the case of enalapril and its maleate salt.

The main decomposition products of Formula (III) and (IV) of enalapril are shown below demonstrating that the decomposition is due to hydrolytic and cyclization processes.

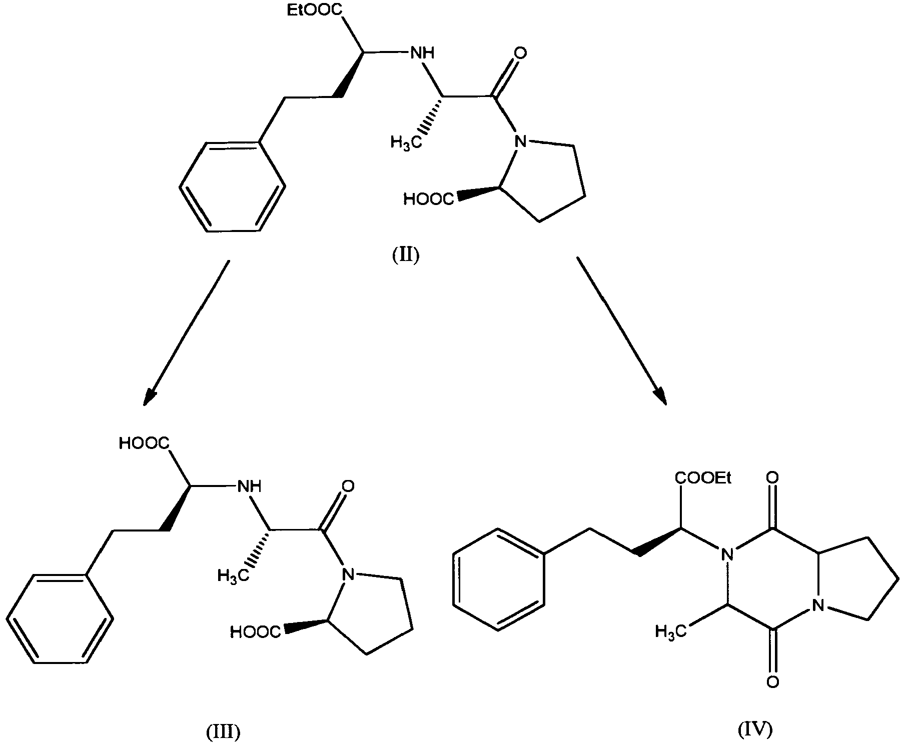

The diketopiperazine or DPK impurity (IV) is the internal cyclization product and the diacid or enalaprilate impurity (III) is the product of ester hydrolysis.

According to the European Patent Application EP 545 194, enalapril was transformed into its sodium salt. The enalapril sodium salt in pharmaceutical preparations is reported to be more stable than the enalapril maleate salt.

EP 099 239 and EP 264 887 suggest ascorbic acid may be used as an antioxidant or color stabilizing agent in case of ACE-inhibitors.

U. S. Pat. No. 5,562,921 discloses that enalapril degrades at a faster rate in the presence of some diluents namely microcrystalline cellulose, dibasic calcium phosphate, and tribasic calcium phosphate; lubricants, namely magnesium stearate and calcium stearate, and disintegrants such as crospovidone, and sodium starch glycolate. The disclosed composition was free or substantially free of microcrystalline cellulose, cellulose derivatives or cellulose polymers, calcium phosphate, disintegrants, and magnesium stearate. At least 50% by weight of the pharmaceutical excipients in the composition were pharmaceutically acceptable water soluble substances such that the composition could dissolve sufficiently rapidly and not require the use of disintegrants. Harris et al., in U.S. Patent 4,743,450, describe the use of stabilizers to minimize the cyclization, hydrolysis, and coloration of ACE inhibitors.

WO 01/19348 and WO 98/26765 both disclose the stabilization of pharmaceutical compositions comprising only enalapril maleate as the pharmaceutically active ingredient.

The fixed combination of lercanidipine HCI and enalapril maleate is marketed by Recordati and Berlin Chemie under the trade names Lercaril®, Carmen ACE®, and Zanipress®. A combination of lercanidipine hydrochloride and enalapril maleate is described in Table 1 of US 2003/0180355 but no process for the preparation of the solid pharmaceutical oral dosage form is disclosed. CN 101836982 describes a pharmaceutical composition comprising lercanidipine and enalapril and sodium bicarbonate.

However, lercanidipine and enalapril are known to be susceptible to degradation and/or oxidation when subjected to unfavorable physical and/or chemical conditions or storage/formulations. Conditions that favorably influence enalapril are usually not optimal for lercanidipine. Also, an optimized combination drug product may call for different release profiles of each of the active substances.

Accordingly, there is an unmet need for providing a dosage form comprising a combination of lercanidipine or a pharmaceutically acceptable salt thereof, preferably lercanidipine hydrochloride, and enalapril or a pharmaceutically acceptable salt thereof, preferably enalapril maleate, in which all active pharmaceutical substances remain stable and wherein the active substances are provided in a formulation providing maximum bioavailability and/or maximum therapeutic or pharmacological response while keeping the manufacturing process as simple as possible and the production costs at a minimum.

The technical problem to be solved by the present invention was also the preparation of a stable and homogenous pharmaceutical solid oral dosage form comprising both lercanidipine or its pharmaceutically acceptable salt and enalapril or a pharmaceutically acceptable salt thereof; preferably lercanidipine HCl and enalapril maleate, with good dissolution profiles.

Under a "homogenous" pharmaceutical solid oral dosage form a pharmaceutical solid oral dosage form is understood that is uniform in composition and structure. The uniformity of tablets is determined according to standard procedures as described in Pharmaceutical Dosage Forms, Second Edition, Volume 2, 1990, H. A. Lieberman, L. Lachman, J. B. Schwarz (editors), Marcel Dekker, Inc., New York and Basel, pp. 321 to 325. In the most restricted meaning a "homogenous" pharmaceutical solid oral dosage form is to be understood as a pharmaceutical solid oral dosage form, i.e. a tablet or a capsule, wherein at least one of the active substances lercanidipine and enalapril or their pharmaceutically acceptable salts is incorporated into a granulate or wherein both lercanidipine and enalapril or their pharmaceutically acceptable salts are together and/or simultaneously incorporated into a granulate.

### Summary of the Invention

The present invention relates in one aspect to a combined homogenous pharmaceutical oral dosage form comprising lercanidipine or its pharmaceutically acceptable salt as the active substance, and enalapril or a pharmaceutically acceptable salt thereof as the active substance, characterized in that the pharmaceutical oral dosage form comprises an organic acid selected from maleic and/or citric acid to stabilize both enalapril and lercanidipine, and their pharmaceutically acceptable salts. Preferably lercanidipine hydrochloride and enalapril maleate are used.

### Description of the Figures

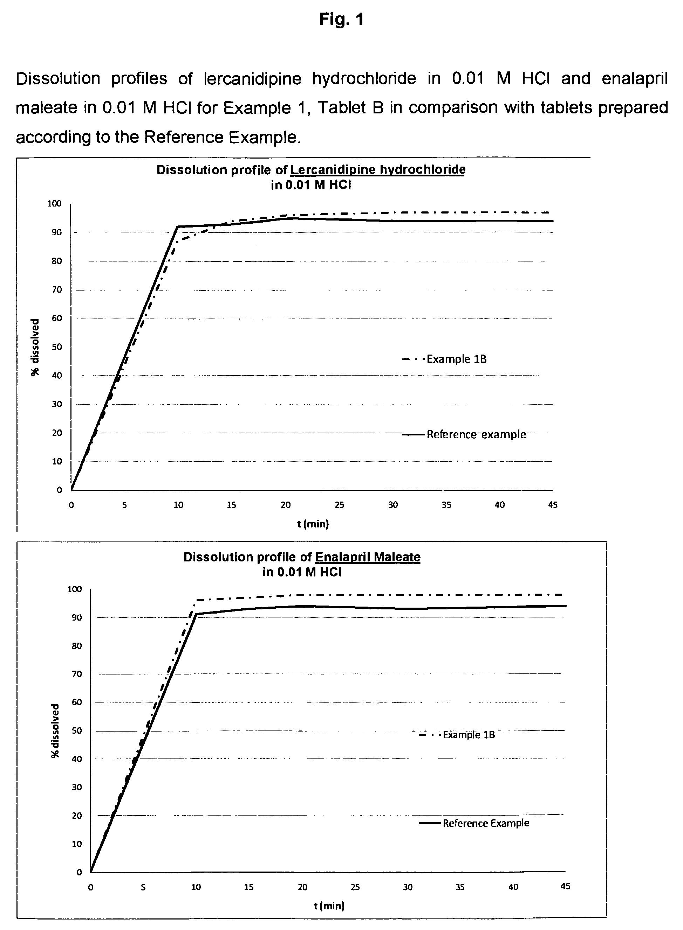

Fig. 1 shows the dissolution profiles of lercanidipine hydrochloride in 0.01 M HCl and enalapril maleate in 0.01 M HCl for Example 1, Tablet B in comparison with tablets prepared according to the Reference Example.

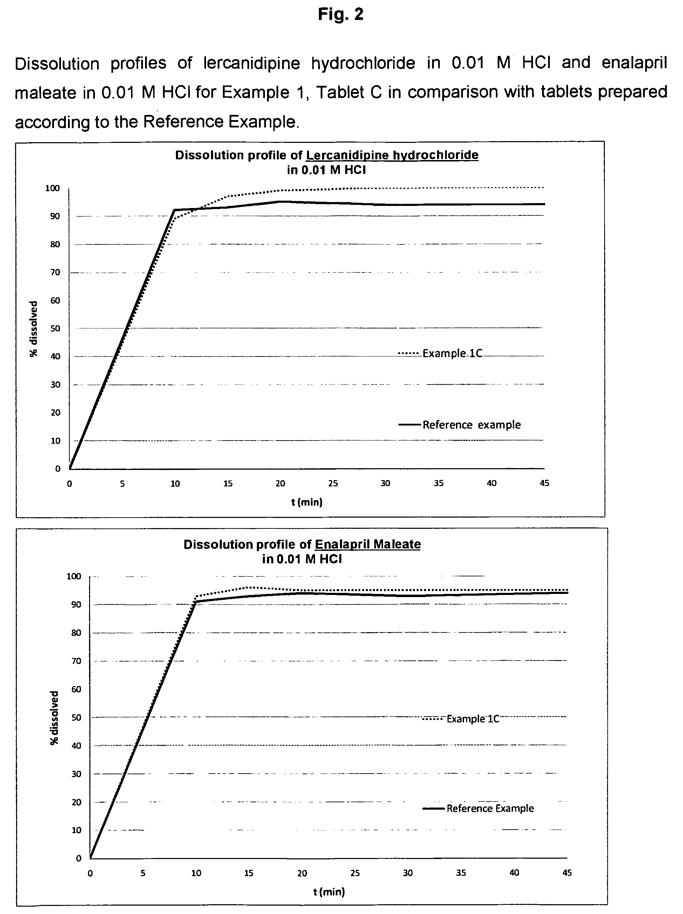

Fig. 2 shows the dissolution profiles of lercanidipine hydrochloride in 0.01 M HCl and enalapril maleate in 0.01 M HCl for Example 1, Tablet C in comparison with tablets prepared according to the Reference Example.

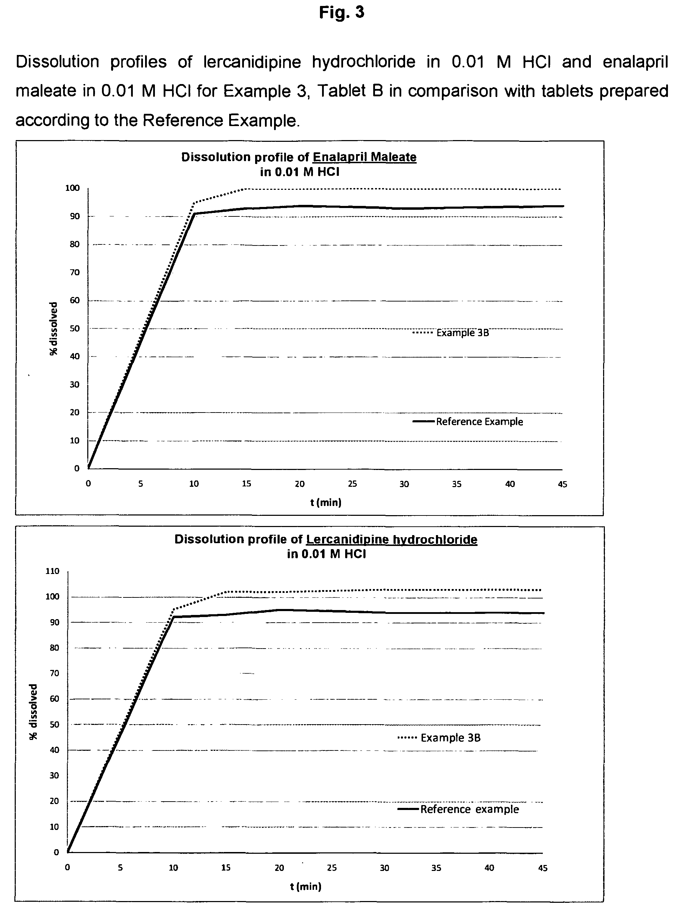

Fig. 3 shows the dissolution profiles of lercanidipine hydrochloride in 0.01 M HCl and enalapril maleate in 0.01 M HCl for Example 3, Tablet B in comparison with tablets prepared according to the Reference Example.

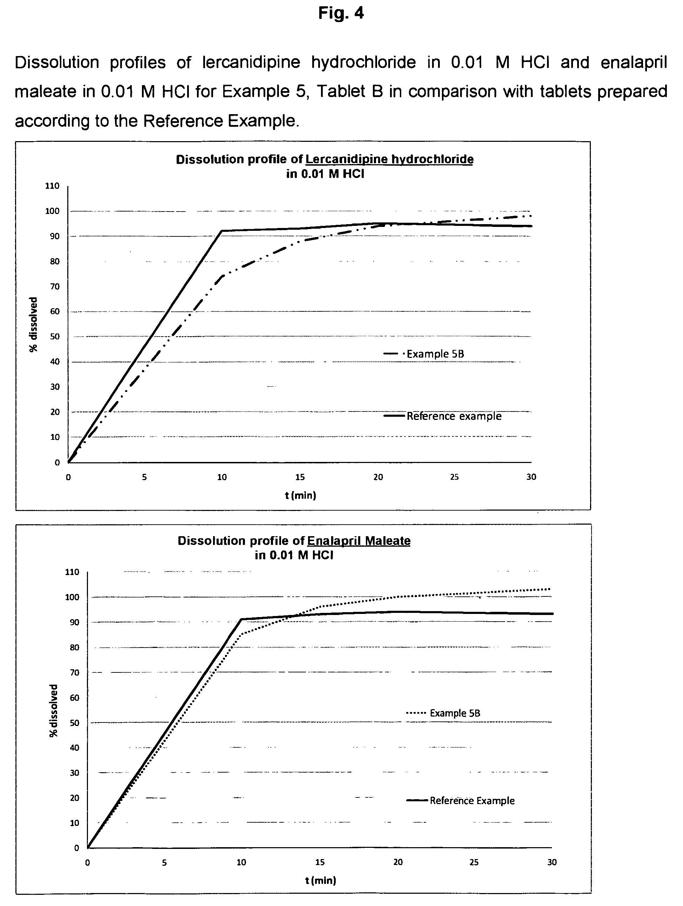

Fig. 4 shows the dissolution profiles of lercanidipine hydrochloride in 0.01 M HCl and enalapril maleate in 0.01 M HCl for Example 5, Tablet B in comparison with tablets prepared according to the Reference Example.

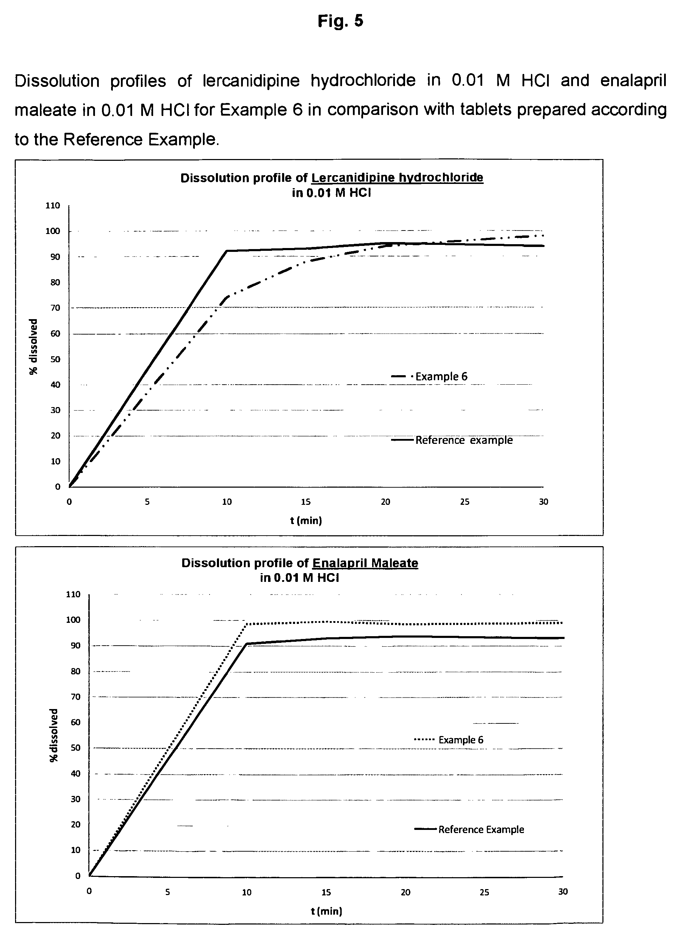

Fig. 5 shows the dissolution profiles of lercanidipine hydrochloride in 0.01 M HCl and enalapril maleate in 0.01 M HCl for Example 6 in comparison with tablets prepared according to the Reference Example

The dissolution profile comparison is based on the dissolution method using apparatus 2 - paddle (Ph.Eur or USP, 50 rpm) filled with 900 mL of 0.01 M HCl Hydrochloric medium at temperature 37°C ± 0.5 °C. Concentrations of dissolved lercanidipine hydrochloride and enalapril maleate were measured by HPLC analysis.

### Detailed Description of the Invention

In a general aspect, the present invention relates to a combined pharmaceutical solid oral dosage form comprising lercanidipine or a pharmaceutically acceptable salt thereof as the active substance, and enalapril or a pharmaceutically acceptable salt thereof as the active substance, and comprising an organic acid, wherein the organic acid is maleic and/or citric acid.

In a preferred embodiment, the present invention relates to a combined homogenous pharmaceutical oral dosage form comprising lercanidipine or its pharmaceutically acceptable salt as the active substance, and enalapril or a pharmaceutically acceptable salt thereof as the active substance, characterized in that the pharmaceutical oral dosage form comprises an organic acid selected from maleic and/or citric acid to stabilize both enalapril and lercanidipine, and their pharmaceutically acceptable salts. Preferably lercanidipine hydrochloride and enalapril maleate are used.

The invention further relates to homogenous pharmaceutical oral dosage forms of the active substances lercanidipine and enalapril, as well the pharmacologically acceptable salts thereof, characterized in that the pharmaceutical oral dosage forms comprise an organic acid selected from maleic and/or citric acid to stabilize both enalapril and lercanidipine, and their pharmaceutically acceptable salts.

In a prefered embodiment, the homogenous pharmaceutical composition is in the form of a granulate, granules, grains, beads or pellets, which are mixed and filled into capsules or sachets or are compressed to tablets by conventional methods.

Under a "homogenous" pharmaceutical solid oral dosage form a pharmaceutical solid oral dosage form is understood that is uniform in composition and structure. The uniformity of tablets is determined according to standard procedures as described in Pharmaceutical Dosage Forms, Second Edition, Volume 2, 1990, H. A. Lieberman, L. Lachman, J. B. Schwarz (editors), Marcel Dekker, Inc., New York and Basel, pp. 321 to 325. In the most restricted meaning a "homogenous" pharmaceutical solid oral dosage form is to be understood as a pharmaceutical solid oral dosage form, i.e. a tablet or a capsule, wherein at least one of the active substances lercanidipine and enalapril or their pharmaceutically acceptable salts is incorporated into a granulate or wherein both lercanidipine and enalapril or their pharmaceutically acceptable salts are together and/or simultaneously incorporated into a granulate.

The expressions »solid pharmaceutical composition«, »pharmaceutical solid oral dosage form" and »pharmaceutical composition« are used interchangeably. The granulate, granules, grains, beads or pellets containing lercanidipine, enalapril and optionally a third active substance are optionally entero-coated or coated with a protective coating. Tablets may be plain, film or sugar coated bisected, embossed, or sustained-release. They can be made in a variety of sizes, shapes and colors. Tablets may be swallowed, chewed or dissolved in the buccal cavity or beneath the tongue.

The expressions "unitary pharmaceutical oral dosage form", "single dosage form", "pharmaceutical solid oral dosage form", "combined pharmaceutical oral dosage form" and "oral pharmaceutical solid oral dosage form" are used interchangeably and can either mean capsules, sachets or tablets comprising both active substances.

Pharmaceutical solid oral dosage forms can generally be prepared by direct compression, dry granulation (slugging or compaction) or wet granulation.

The wet granulation method is used to convert a powder mixture into granules having suitable flow and cohesive properties for compression. The procedure consists of mixing the powders in a suitable blender followed by adding the granulating solution under shear to the mixed powders to obtain a granulation. The damp mass is then screened through a suitable screen and dried by tray drying or fluidized bed drying. Alternately, the wet mass may be dried and passed through a mill. The overall process includes weighing, dry powder blending, wet granulating, drying, milling, blending lubrication and compression.

When preparing the homogenous pharmaceutical composition by granulation, preferably wet granulation, the granulate may either be prepared by:
i.) dissolving the pharmaceutically active substances in water, a suitable organic solvent, or a molten binder, or a mixture thereof, optionally with some of the pharmaceutically inactive excipients used, and granulating the remaining pharmaceutically inactive excipients therewith, or
ii.) dissolving a part of the pharmaceutically active substances in water, a suitable organic solvent, or a molten binder, or a mixture thereof, optionally with some of the pharmaceutically inactive excipients used, and granulating the remaining pharmaceutically active and inactive excipients therewith, or
iii.) optionally dissolving some of the pharmaceutically inactive excipients in water, a suitable organic solvent, or a molten binder, and granulating the pharmaceutically active substance and the pharmaceutically inactive excipients therewith.

The organic acid used according to the invention may be applied either in the granulation liquid or as a component of the pharmaceutically inactive excipients.

The liquid applied for the preparation of the granulation liquid may be chosen among water or an organic solvent such as alcohols with 1 to 4 carbon atoms like absolute ethanol, concentrated ethanol (96 vol%), methanol, isopropanol, ketones such as acetone or esters such as ethylacetate, or a mixture of an organic solvent and water. Preferably water is used and when a mixture of an organic solvent and water is applied, the preferred solvent mixture is the mixture of ethanol and water. The volume to volume ratio between ethanol and water may range from 1:15 to 15:1.

The granulate of enalapril or its pharmaceutically acceptable salt, preferably enalapril maleate, is preferably prepared by the method iii.) wherein the active substances and optionally inactive pharmaceutical excipients are granulated with water, an organic solvent or a mixture of an organic solvent and water, together with one or more of the pharmaceutically acceptable inactive excipients, in particular the organic acid, preferably maleic acid or citric acid together forming the granulation liquid.

The granulate of the homogenous pharmaceutical composition of the present invention is prepared using state of the art granulators such as a high shear granulator, low shear granulator, or a fluid bed granulator. Preferably a fluid bed granulator is applied. The obtained granules are dried to achieve loss on drying (LOD) determined by halogen dryer Mettler HR73 (20 minutes at 85 °C) of less than 4 wt %, preferably below 2 wt %, most preferably below 1 wt %, and optionally sieved through a sieve with sieve openings 1.00 mm. The drying of the granulate is preferably carried out at a temperature of 20°C to 90°C, for 1 to 80 minutes.

The compression of the granulate to tablet cores can be carried out in a conventional tabletting machine, e.g. in an eccentric tabletting machine or a rotary compression machine, at a compression between 1 to 40 kN. The tablet cores may vary in shape and be, for example, round, oval, oblong, cylindrical or any other suitable shape, and may also vary in size depending on the concentration of the therapeutic agents. A characteristic of tablets according to the invention is their small size having regard to the amount of active agent contained therein.

In a preferred embodiment of the present invention, the invention provides a method for preparing a homogenous pharmaceutical oral dosage form of the active substances lercanidipine and enalapril, as well the pharmacologically acceptable salts thereof, further comprising an organic acid selected from maleic and/or citric acid to stabilize both enalapril and lercanidipine, and their pharmaceutically acceptable salts, which method comprisses the steps of:
i.) dissolving or suspending the organic acid in a liquid to prepare a granulation liquid or suspension,
ii.) granulating therewith a mixture of both active ingredients lercanidipine and enalapril or their pharmaceutically acceptable salts, optionally together with other pharmaceutically acceptable excipients or part of pharmaceutically acceptable excipients used,
iii.) drying the granulate at a temperature of 20 to 90 °C for 1 to 80 minutes, preferably to achieve a loss on drying of less than 4 wt %, preferably below 2 wt %, most preferably below 1 wt %,
iv.) admixing other pharmaceutically acceptable excipients or part of pharmaceutically acceptable excipients used to the granulate prepared under iii.),
v.) compressing the mixture obtained under iv.) into a tablet at a pressure of 1 to 40 kN, and
vi.) optionally coating the tablet obtained under v.).

In another preferred embodiment of the present invention, the invention provides a method for preparing a homogenous pharmaceutical oral dosage form of the active substances lercanidipine and enalapril, as well the pharmacologically acceptable salts thereof, further comprising an organic acid selected from maleic and/or citric acid to stabilize both enalapril and lercanidipine, and their pharmaceutically acceptable salts, which method comprises the steps of:
i.) dissolving or suspending the organic acid and optionally other pharmaceutically acceptable excipients in a liquid to prepare a granulation liquid or suspension,
ii.) granulating therewith the active substance enalapril or its pharmaceutically acceptable salt optionally together with other pharmaceutically acceptable excipients or part of pharmaceutically acceptable excipients used,
iii.) drying the granulate at a temperature of 20 to 90 °C for 1 to 80 minutes, preferably to achieve a loss on drying of less than 4 wt %, preferably below 2 wt %, most preferably below 1 wt %,
iv.) mixing the dried granulate obtained under iii.) with the active substance lercanidipine or its pharmaceutically acceptable salt and other pharmaceutically acceptable excipients or part of pharmaceutically acceptable excipients used,
v.) compressing the mixture obtained under iv.) into a tablet at a pressure of 1 to 40 kN,
vi.) optionally coating the tablet obtained under v.).

The method of preparation and type of excipients are selected as to give the tablet formulation the desired physical characteristics that allow a rapid compression of the tablets. After compression, the tablets must have a number of additional attributes, such as appearance, hardness, disintegrating ability and an acceptable dissolution profile. Choice of fillers and other excipients will depend on the chemical and physical properties of the drug, behavior of the mixture during processing and the properties of the final tablets.

The homogenous pharmaceutical compostion of the present invention comprises lercanidipine or its pharmaceutically acceptable salt, preferably lercanidipine hydrochloride, wherein the d₅₀ particle size is preferably less than 50 micrometers, more preferably less than 30 micrometers and most preferably less than 20 micrometers.

The d₅₀ equivalent spherical volume diameter is a statistical representation of the 50% point on a cumulative frequency plot. As indicated, the d₅₀ equivalent sphere volume diameter of the particles of the active substance are evaluated using a Malvern Mastersizer Laser Scattering Particle Size Distribution Analyzer or other such equipment recognized by those skilled in the art. Using such instrument values for a suspension of the particles of unknown size in Isopar L (A=1) are obtained.

If desired, lercanidipine or its pharmaceutically aceptable salt may be milled to reduce the size of the particles as required.

The active substance and the additives can be milled either individually or together to particle sizes from about 0.1 micrometers to 500 micrometers, preferably 1.0 micrometers to 200 micrometers. At least 90 % of the particles of both active substances and the additives are present in these ranges. Particles of this size are obtained by conventional comminution methods, e.g. grinding in an air jet mill, hammer and screen mill, fine impact mill, ball mill or vibrator mill.

Micronisation is preferably effected by known methods using an ultrasonic disintegrator, e.g. of the BRANSON Sonifier type, or by stirring a suspension with a high speed agitator, for example with a stirrer of the HOMOREX type.

The ground particles may optionally at this stage be sieved and mixed according to known methods.

Any polymorphic form of lercanidipine hydrochloride or any other pharmaceutically acceptable salt of lercanidipine known to the person skilled in the art may be applied, e.g. amorphous lercanidipine hydrochloride as prepared according to EP 153016; form I as described in EP 824517, EP 1600441, EP 2036890, or EP 839036, form II as disclosed by WO 03/014084, form III or form IV as disclosed by EP 1423367, form V as described by EP 1940790 and WO2010000545, or forms A and B, as prepared by EP 1432683. Preferably amorphous lercanidipine hydrochloride (either in purely amorphous form or comprising 1 wt% to 5 wt% of a crystalline form), form V, or form I lercanidipine hydrochloride is applied.

The average particle size (d₅₀) of enalapril or its pharmaceutically acceptable salts, preferably enalapril maleate, is less than 600 µm, more preferably less than 500 µm, e.g. between 10 nanometers and 500 µm, most preferably in the range of 0.2 µm to 150 µm, as measured by laser light diffraction. Any polymorphic form of enalapril maleate or any other pharmaceutically acceptable salt of enalapril known to the person skilled in the art may be applied, preferably amorphous enalapril maleate or crystalline forms I and/or II.

The organic acid used for the preparation of the combined homogenous pharmaceutical oral dosage form of the present invention comprising lercanidipine or its pharmaceutically acceptable salt, and enalapril or a pharmaceutically acceptable salt thereof as the active substance, is chosen from the group of citric acid and maleic acid. Optionally a combination of these organic acids may be used, either both of them used in the preparation of the granulation liquid or at least one of them used in the preparation of the granulation liquid under i.) and the second or more used as additional pharmaceutically acceptable excipients under ii.) and/or iv.). Most preferably, maleic acid is used.

Lercanidipine and enalapril are sensitive to water and to oxidizing agents, so when including them into pharmaceutical formulations, care should be taken that the pharmaceutically acceptable excipient(s) used do not contain a non-negligible quantity of them. Lercanidipine is and its pharmaceutically acceptable salts, and in particular lercanidipine hydrochloride, are very light-sensitive and should therefore be protected from sunlight during the manufacturing process of the active substance itself, as well as during the preparation and packaging of the pharmaceutical composition and final product, i.e. tablet or capsule, comprising it.

In the present context the terms "pharmaceutically acceptable inactive excipient", "inactive excipient" or "pharmaceutically acceptable excipient" are intended to denote any material, which is inert in the sense that it substantially does not have any therapeutic and/or prophylactic effect *per se*. Such excipients may be added with the purpose of making it possible to obtain a pharmaceutical composition, which have acceptable technical properties. The solid oral dosage form according to the invention may contain one or more pharmaceutically acceptable excipients. Examples of suitable excipients for use in a composition or solid dosage form according to the invention include fillers, diluents, disintegrants, binders, stabilizers, lubricants etc. or mixtures thereof. As the composition or solid dosage form according to the invention may be used for different purposes, the choice of excipients is normally made taken such different uses into considerations. Other pharmaceutically acceptable excipients for suitable use are e.g. preservatives, antioxidants, buffering agents, chelating agents, coloring agents, complexing agents, emulsifying and/or solubilizing agents, flavors and perfumes, humectants, sweetening agents, wetting agents etc.

The amount of each type of additive employed, e.g. glidant, binder, disintegrant, filler or diluent and lubricant may vary within ranges conventional in the art. Thus for example, the amount of glidant may vary within a range of from 0.1 to 10% by weight, in particular 0.1 to 5% by weight, e.g. 0.1 to 0.5% by weight; the amount of binder may vary within a range of from about 10 to 45% by weight, e.g. 20 to 30% by weight; the amount of disintegrant may vary within a range of from 2 to 20% by weight, e.g. 15% by weight; the amount of filler or diluent may vary within a range of from 15 to 40% by weight; whereas the amount of lubricant may vary within a range of from 0.1 to 5.0% by weight.

The absolute amounts of each additive and the amounts relative to other additives is similarly dependent on the desired properties of the solid oral dosage form and may also be chosen by the skilled artisan by routine experimentation without undue burden. For example, the solid oral dosage form may be chosen to exhibit accelerated and/or delayed release of the active agent with or without quantitative control of the release of active agent.

The oral dosage form according to the invention preferably comprises 0.05 to 15.0, in particular 0.05 to 7.0, preferably 0.1 to 5.0, more preferably 0.1 to 3.0 and most preferably 0.2 to 2.5 % by weight of the organic acid.

Examples of suitable fillers, diluents and/or binders include lactose (e.g. spray-dried lactose, a-lactose, b-lactose, Tabletose®, various grades of Pharmatose®, Microtose® or Fast-Floe®), microcrystalline cellulose (various grades of Avicel®, Elcema®, Vivacel®, Ming Tai® or Solka-Floc®), hydroxypropylcellulose, L-hydroxypropylcellulose (low substituted), hydroxypropyl methylcellulose (HPMC) (e.g., Methocel E, F and K, Metolose SH of Shin- Etsu, Ltd, such as, e.g. the 4,000 cps grades of Methocel E and Metolose 60 SH, the 4,000 cps grades of Methocel F and Metolose 65 SH, the 4,000, 15,000 and 100,000 cps grades of Methocel K; and the 4,000, 15,000, 39,000 and 100,000 grades of Metolose 90 SH), methylcellulose polymers (such as, e.g., Methocel A, Methocel A4C, Methocel A15C, Methocel A4M), hydroxyethylcellulose, sodium carboxymethylcellulose, carboxymethyl-hydroxylethylcellulose and other cellulose derivatives, sucrose, agarose, sorbitol, mannitol (e.g. Pearlitol 50C), dextrins, maltodextrins, starches or modified starches (including potato starch, maize starch and rice starch), calcium phosphate (e.g., basic calcium phosphate, calcium hydrogen phosphate, dicalcium phosphate hydrate), calcium sulfate, calcium carbonate, sodium alginate, collagen etc.

Specific examples of diluents are e.g., calcium carbonate, dibasic calcium phosphate, tribasic calcium phosphate, calcium sulfate, microcrystalline cellulose, powdered cellulose, dextrans, dextrin, dextrose, fructose, kaolin, lactose, mannitol, sorbitol, starch, pregelatinized starch, sucrose, sugar etc.

Specific examples of disintegrants are e.g. alginic acid or alginates, microcrystalline cellulose, hydroxypropyl cellulose and other cellulose derivatives, croscarmellose sodium (Ac-di-sol), crospovidone, polacrillin potassium, sodium starch glycolate, starch, pregelatinized starch, carboxymethyl starch (e.g. Primogel® and Explotab®) etc.

Preferably mannitol, microcrystalline cellulose and lactose are used as fillers, most preferably mannitol and/or lactose is used.

Specific examples of binders are e.g., acacia, alginic acid, agar, calcium carrageenan, sodium carboxymethylcellulose, microcrystalline cellulose, dextrin, ethylcellulose, gelatin, liquid glucose, guar gum, hydroxypropyl methylcellulose, hydroxypropyl cellulose, methylcellulose, pectin, PEG, povidone, pregelatinized starch etc. Preferably, povidone or hydroxypropyl cellulose is used as binder.

Glidants and lubricants may also be included in the first and/or the second composition. Examples include stearic acid, magnesium stearate, calcium stearate or other metalic stearate, talc, waxes and glycerides, light mineral oil, PEG, glyceryl behenate, colloidal silica, hydrogenated vegetable oils, corn starch, sodium stearyl fumarate, polyethylene glycols, alkyl sulfates, sodium benzoate, sodium acetate etc.

As disintegrants one can particularly mention sodium starch glycolate, CMC-Ca, CMC-Na, crosslinked PVP (Crospovidone, Polyplasdone of Kollidon XL), Alginic acid, sodium alginate and guar gum, most preferably sodium starch glycolates, crosslinked PVP, Crospovidone, crosslinked CMC and Ac-Di-Sol.

Other excipients which may be included in a composition or solid dosage form of the invention are e.g., flavoring agents, coloring agents, taste-masking agents, pH-adjusting agents, buffering agents, preservatives, stabilizing agents, anti-oxidants, wetting agents, humidity-adjusting agents, surface-active agents (e.g. Polysorbate 80/Tween 80), suspending agents, absorption enhancing agents, agents for modified release etc.

Other additives in a composition or a solid dosage form according to the invention may be antioxidants like e.g. ascorbic acid, ascorbyl palmitate, butylated hydroxyanisole, butylated hydroxytoluene, citric acid, hypophosphorous acid, monothioglycerol, potassium metabisulfite, propyl gallate, sodium formaldehylde sulfoxylate, sodium metabisulfite, sodium thiosulfate, sulfur dioxide, tocopherol, tocopherol acetate, tocopherol hemisuccinate, TPGS or other tocopherol derivatives, etc.

A composition or solid dosage form according to the invention may also include one or more surfactants or substances having surface-active properties. It is contemplated that such substances are involved in the wetting of the slightly soluble active substance and thus, contributes to improved solubility characteristics of the active substance. Suitable surfactants for use in a composition or a solid dosage form according to the invention are surfactants such as, e.g., hydrophobic and/or hydrophilic surfactants as those disclosed in WO 00/50007 in the name of Lipocine, Inc. Specific examples of suitable surfactants are polyethoxylated fatty acids such as, e.g., fatty acid mono- or diesters of polyethylene glycol or mixtures thereof such as, e.g., mono - or diesters of polyethylene glycol with lauric acid, oleic acid, stearic acid, myristic acid, ricinoleic acid, and the polyethylene glycol may be selected from PEG 4, PEG 5, PEG 6, PEG 7, PEG 8, PEG 9, PEG 10, PEG 12, PEG 15, PEG 20, PEG 25, PEG 30, PEG 32, PEG 40, PEG 45, PEG 50, PEG 55, PEG 100, PEG 200, PEG 400, PEG 600, PEG 800, PEG 1000, PEG 2000, PEG 3000, PEG 4000, PEG 5000, PEG 6000, PEG 7000, PEG 8000, PEG 9000, PEG 1000, PEG 10,000, PEG 15,000, PEG 20,000, PEG 35,000, polyethylene glycol glycerol fatty acid esters, i.e. esters like the above-mentioned but in the form of glyceryl esters of the individual fatty acids; glycerol, propylene glycol, ethylene glycol, PEG or sorbitol esters with e.g., vegetable oils like e.g., hydrogenated castor oil, almond oil, palm kernel oil, castor oil, apricot kernel oil, olive oil, peanut oil, hydrogenated palm kernel oil and the like, polyglycerized fatty acids like e.g., polyglycerol stearate, polyglycerol oleate, polyglycerol ricinoleate, polyglycerol linoleate, propylene glycol fatty acid esters such as, e.g., propylene glycol monolaurate, propylene glycol ricinoleate and the like, mono- and diglycerides like e.g. glyceryl monooleate, glyceryl dioleae, glyceryl mono- and/or dioleate, glyceryl caprylate, glyceryl caprate etc.; sterol and sterol derivatives; polyethylene glycol sorbitan fatty acid esters (PEG-sorbitan fatty acid esters) such as esters of PEG with the various molecular weights indicated above, and the various Tween® series (from ICI America, Inc.); polyethylene glycol alkyl ethers such as, e.g., PEG oleyl ether and PEG lauryl ether; sugar esters like e.g. sucrose monopalmitate and sucrose monolaurate; polyethylene glycol alkyl phenols like e.g. the Triton® X or N series (Union Carbide Chemicals & Plastics Technology Corporation); polyoxyethylene-polyoxypropylene block copolymers such as, e.g., the Pluronic® series from BASF Aktiengesellschaft, the Synperonic® series from ICI America, Inc., Emkalyx , Lutrol® from BASF Aktiengesellschaft, Supronic etc. The generic term for these polymers is "poloxamers" and relevant examples in the present context are Poloxamer 105, 108, 122, 123, 124, 181 , 182, 183, 184, 185, 188, 212, 215, 217, 231 , 234, 235, 237, 238, 282, 284, 288, 331 , 333, 334, 335, 338, 401, 402, 403 and 407; sorbitan fatty acid esters like the Span® series (from ICI) or Arlacel® series (from ICI) such as, e.g., sorbitan monolaurate, sorbitan monopalmitate, sorbitan monooleate, sorbitan monostearate etc.; lower alcohol fatty acid esters like e.g., oleate, isopropyl myristate, isopropyl palmitate etc.; ionic surfactants including cationic, anionic and zwitterionic surfactants such as, e.g., fatty acid salts, bile salts, phospholipids, phosphoric acid esters, carboxylates, sulfates and sulfonates etc.

When a surfactant or a mixture of surfactants is present in a composition or a solid dosage form of the invention, the concentration of the surfactant(s) is normally in a range of from about 0.1 - 80% w/w such as, e.g., from about 0.1 to about 20% w/w, from about 0.1 to about 15% w/w, from about 0.5 to about 10% w/w, or alternatively, from about 0.10 to about 80% w/w such as, e.g. from about 10 to about 70% w/w, from about 20 to about 60% w/w or from about 30 to about 50% w/w.

In a specific aspect of the invention, the at least one of the one or more pharmaceutically acceptable excipient is selected from the group consisting of silica acid or a derivative or salt thereof including silicates, silicon dioxide and polymers thereof; magnesium aluminosilicate and/or magnesium atuminometasilicate, bentonite, kaolin, magnesium trisilicate, montmorillonite and/or saponite.

In the present invention, the amount of enalapril administered to a patient in the combination therapy will be preferably within the range of 5 to 40 mg per day in a single or two divided doses. More preferably, the amount of enalapril will be 5-20 mg per day. The amount of lercanidipine will be preferably within the range of 5-40 mg, more preferably, 10-20 mg. The preferred combinations are (i) 5 mg of enalapril and 5 mg of lercanidipine, (ii) 10 mg of enalapril and 5 mg of lercanidipine, (iii) 10 mg of enalapril and 10 mg of lercanidipine, (iv) 20 mg enalapril and 10 mg lercanidipine, and (v) 20 mg of enalapril and 20 mg of lercanidipine; but amounts may need to be optimized according to the needs of particular patient sub-populations depending on whether they are responders, partial reponders, nonresponders, or naive to lercanidipine and/or enalapril monotherapy at a tolerated dose (In the case of naive patients, the starting amounts of the combination may be even smaller that the indicated dose, e.g., 2.5 mg of enalapril).

In a most preferred embodiment of the invention there is provided a homogenous single solid oral dosage form, preferably coated, comprising a fixed dose combination selected from the group consisting of lercanidipine 10 mg and enalapril 10 mg; and lercanidipine 10 mg and enalapril 20 mg.

The composition or the oral solid dosage form according to the invention may be coated with a film coating, an enteric coating, a modified release coating, a protective coating, an anti-adhesive coating etc.

A solid dosage form according to the invention may also be coated in order to obtain suitable properties e.g. with respect to release of the active substance. The coating may be applied on single unit dosage forms (e.g. tablets, capsules) or it may be applied on a polydepot dosage form or on its individual units.

Suitable coating materials are e.g. methylcellulose, hydroxypropylmethyl- cellulose, hydroxypropylcellulose, acrylic polymers, ethylcellulose, cellulose acetate phthalate, polyvinyl acetate phthalate, hydroxypropyl methylcellulose phthalate, polyvinylalcohol, sodium carboxymethylcellulose, cellulose acetate, cellulose acetate phthalate, gelatin, methacrylic acid copolymer, polyethylene glycol, shellac, sucrose, titanium dioxide, camauba wax, microcrystalline wax, zein.

Plasticizers and other ingredients may be added in the coating material. The same or different active substance may also be added in the coating material.

The homogenous solid oral dosage form of the present invention can be packed into primary packaging having decreased permeability for water vapor such as high density polyethylene (HDPE) containers with closure, such as polypropylene (PP) closure and with or without a desiccant; aluminium foil blisters or polychlorotrifluoroethylene (Aclar®) blisters or any other suitable water vapour low-permeable packaging such as blisters made of multilayer polymeric foil where different polymeric materials are combined into a single foil. The present invention further on relates to a stabilized pharmaceutical product comprising a package and packaging method that utilizes an adsorbent material, such as a molecular sieve, that adsorbs or absorbs moisture in the inner local environment of an impermeable package, so as to prevent formation of hydrolysis products which result from chemical reactions between the active substance(s). The adsorbent material can be located in a variety of places. For example, the adsorbent material can be situated in the sealed package, or within in a porous sachet that, in turn, is located within the sealed package. Naturally, numerous adsorbent materials have applications in a stable pharmaceutical product or a method of the present invention, including a molecular sieve, an activated clay, charcoal, an activated alumina, silica, a zeolite, a bauxite, or any mixture of these materials, to name only a few. An effective amount of the adsorbent material used in a stable pharmaceutical product or in a method of the present invention is that amount sufficient to reduce or eliminate the formation of degradation products. One of ordinary skill can readily determine this amount for a particular embodiment of the present invention using routine laboratory techniques. Moreover, a sealed package of a stable pharmaceutical product or a method of the present invention can be produced from a variety of materials, e.g. metal, glass, plastic, etc. Similarly, the shape of a sealed package can vary. Examples of such shapes include, but certainly are not limited to bottle, a bag, a drum box, and an irregularly shaped container. The sealing of a package of a stable pharmaceutical product or a method of the present invention can be accomplished in a variety of ways. More specifically, heat-sealing, gluing, welding, brazing, mechanical closures, mechanical clamps, or compression can hermetically seal a sealed package of a stable pharmaceutical product of the present invention.

Due to the light sensitivity of lercanidipine and its pharmaceutically acceptable salts, in particular lercanidipine hydrochloride, light impermeable packaging such as e.g. aluminium foil blisters is preferably used. During the packaging and/or storing controlled conditions with low humidity can be used. Additionally inert gases such as nitrogen, argon or helium; or vacuum can be used during the manufacturing of the solid oral dosage forms, the manufacturing of the homogenous pharmaceutical compositions as well as during their packaging to assure inert atmosphere around the solid oral dosage form, such as tablet or capsule, in the sealed primary packaging. Inert atmosphere according to present invention means that the concentration of oxygen in the atmosphere around the solid dosage form such as tablet containing lercanidipine and enalapril or their salts, in particular lercanidipine hydrochloride and enalapril maleate, packed in the primary packaging is less than 10 vol%, preferably less than 5 vol% and most preferably less than 2 vol%. The concentration of oxygen in the atmosphere surrounding the tablet can be determined by gas chromatography.

The lercanidipine and enalapril impurities were determined by high performance liquid chromatography (HPLC) by the following method:
- **Apparatus:**: Liquid chromatograph with a variable UV - detector.

### Chromatographic conditions:

**Column:** X Bridge C18 or an equivalent column C18
**Mobile phase:** gradient elution

| time (min) | %A | %B |
|---|---|---|
| 0 | 97 | 3 |
| 7 | 97 | 3 |
| 40 | 40 | 60 |
| 50 | 40 | 60 |
| 50.1 | 97 | 3 |
| 55 | 97 | 3 |

| | | |
|---|---|---|
| A: 0.01 M Sodium dihydrogen phosphate B: Acetonitrile | | |

**Flow rate:** about 1.0 ml per min
**Detection:** UV, wavelength 215 nm for enalapril maleate and wavelength 240 nm for lercanidipine
**Injection:** 15 µL

In particular, the present invention provides the following items:
1.) A combined homogenous pharmaceutical oral dosage form comprising lercanidipine or its pharmaceutically acceptable salt as the active substance, and enalapril or a pharmaceutically acceptable salt thereof as the active substance, characterized in that the pharmaceutical oral dosage form comprises an organic acid selected from maleic and/or citric acid to stabilize both enalapril and lercanidipine, and their pharmaceutically acceptable salts.
2.) A combined pharmaceutical oral dosage form according to item 1, characterized in that lercanidipine hydrochloride and enalapril maleate are used.
3.) A combined pharmaceutical oral dosage form according to item 1 or 2, wherein at least one of the active substances lercanidipine and enalapril or their pharmaceutically acceptable salts is incorporated into a granulate or wherein both lercanidipine and enalapril or their pharmaceutically acceptable salts are together and/or simultaneously incorporated into a granulate.
4.) A combined pharmaceutical oral dosage form according to items 1 to 3, characterized in that amorphous lercanidipine hydrochloride (either in purely amorphous form or comprising 1 wt% to 5 wt% of a crystalline form), form V, or form I lercanidipine hydrochloride is used.
5.) A combined pharmaceutical oral dosage form according to items 1 to 4, characterized in that it is in the form of a granulate, granules, grains, beads or pellets, which are mixed and filled into capsules ar sachets or are compressed to tablets.
6.) A combined pharmaceutical oral dosage form according to items 1 to 5, characterized in that it comprises mannitol and/or lactose as fillers.
7.) A method for preparing a combined homogenous pharmaceutical oral dosage form according to items 1 to 6, characterized in comprises the steps of:
   i.) dissolving the pharmaceutically active substances in water, a suitable organic solvent, or molten binder, or a mixture thereof, optionally with some of the pharmaceutically inactive excipients used, and granulating the remaining pharmaceutically inactive excipients therewith, or
   ii.) dissolving a part of the pharmaceutically active substances in water, a suitable organic solvent, or molten binder, or a mixture thereof, optionally with some of the pharmaceutically inactive excipients used, and granulating the remaining pharmaceutically active and inactive excipients therewith, or
   iii.) optionally dissolving some of the pharmaceutically inactive excipients in water, a suitable organic solvent, or molten binder, or a mixture thereof, and granulating the pharmaceutically active substance and the pharmaceutically inactive excipients therewith.
8.) A method for preparing a combined pharmaceutical oral dosage form according to items 1 to 6, characterized in comprises the steps of:
   i.) dissolving or suspending the organic acid in a liquid to prepare a granulation liquid or suspension,
   ii.) granulating a mixture of both active ingredients lercanidipine and enalapril or their pharmaceutically acceptable salts, optionally together with other pharmaceutically acceptable excipients or part of pharmaceutically acceptable excipients used,
   iii.) drying the granulate at a temperature of 20 to 90°C for 1 to 80 minutes,
   iv.) admixing other pharmaceutically acceptable excipients or part of pharmaceutically acceptable excipients used to the granulate prepared under iii.),
   v.) compressing the mixture obtained under iv.) into a tablet at a pressure of 1 to 40 kN,
   vi.) optionally coating the tablet obtained under v.).
9.) A method for preparing a combined pharmaceutical oral dosage form according to items 1 to 6, characterized in that it comprises the steps of:
   i.) dissolving or suspending the organic acid and optionally other pharmaceutically acceptable excipients in a liquid to prepare a granulation liquid or suspension,
   ii.) granulating the active substance enalapril or its pharmaceutically acceptable salt optionally together with other pharmaceutically acceptable excipients or part of pharmaceutically acceptable excipients used,
   iii.) drying the granulate at a temperature of 20 to 90 °C for 1 to 80 minutes,
   iv.) mixing the dried granulate obtained under iii.) with the active substance lercanidipine or its pharmaceutically acceptable salt and other pharmaceutically acceptable excipients or part of pharmaceutically acceptable excipients used,
   v.) compressing the mixture obtained under iv.) into a tablet at a pressure of 1 to 40 kN,
   vi.) optionally coating the tablet obtained under v.).
10.) A method according to any of items 7 or 9, characterized in that the combined pharmaceutical oral dosage form is prepared by fluid bed granulation.
11.) A method according to any of items 7 to 10, characterized in that the combined pharmaceutical oral dosage form is prepared by aqueous granulation.
12.) A combined homogenous pharmaceutical oral dosage form according to any of items 1 to 6 or prepared by the methods according to any of items 7 to 11, characterized in that the combined homogenous pharmaceutical composition comprises lercanidipin or its pharmaceutically acceptable salt, preferably lercanidipine hydrochloride, wherein the d50 particle size is less than 50 micrometers, preferably less than 30 micrometers and most preferably less than 20 micrometers.

The present invention is illustrated by the following non-limiting examples.

### Examples

### Reference Example

Sodium hydrogen carbonate, enalapril maleate and povidone were dissolved in water. With the obtained granulation liquid lercanidipine hydrochloride (amorphous), lactose and a part of sodium starch glycolate were granulated. The obtained granules were dried. The dried granules were mixed with microcrystalline cellulose, the second part of sodium starch glycolate and magnesium stearate and compressed into tablets. The obtained tablets were coated with the coating agent based on hydroxypropylmethyl cellulose.

| **Excipients and active substances** | **Function** | **Tablet A** |
|---|---|---|
| Enalapril maleate | active | 20.0 |
| Sodium hydrogen carbonate | stabilizer | 10.2 |
| Lercanidipine HCl | active | 10.0 |
| Povidone | binder | 8.0 |
| Lactose crystalline | filler | 235.3 |
| Sodium starch glycolate | disintegrant | 15.0 |
| Microcrystalline cellulose | filler | 80.0 |
| Sodium starch glycolate | disintegrant | 25.0 |
| Magnesium stearate | lubricant | 4.0 |
| **Total tablet core** | | **407.5** |
| | | |
| Coating agent | coating agent | 10.0 |
| **Total tablet** | | **417.5** |

**Stability results of Tablets A prepared according to the Reference Example**

| | Stability conditions | Enalaprilate (III) (%) | DKP(IV) (%); EM | Impurity with MH⁺ 331.1 Da (C₁₆H₁₅O₆N₂); resulting from degradation of lercanidipine (%) | Impurity with MH⁺ 418.2 Da (C₂₁H₂₈O₆N₃); resulting from degradation of lercanidipine (%) | Impurity with MH⁺ 598.3 Da (C₃₅H₄₀O₆N₃); resulting from degradation of lercanidipine (%) | Impurity with MH⁺ 610.3 Da = LRCNA (C₃₆H₄₀O₆N₃); resulting from degradation of lercanidipine (%) |
|---|---|---|---|---|---|---|---|
| Reference Example, Tablet A | t₀ | 0.07 | 0.02 | 0.01 | 0.04 | 0.06 | 0.06 |
| | 50°C-closed - 7 days | 1.21 | 4.33 | 0.22 | 0.48 | 0.92 | 0.76 |

### Example 1

Binder (Hydroxy propylcellulose or povidone) and maleic acid were dissolved in water. With the obtained granulation liquid, enalapril maleate, lercanidipine hydrochloride (polymorph V), part of sodium starch glycolate and a part of mannitol were granulated. The obtained granules were dried. The dried granules were mixed with the second part of sodium starch glycolate, the second part of mannitol, and sodium stearyl fumarate, and compressed into tablets. The obtained tablets were coated with the coating agent based on hydroxypropylmethyl cellulose.

| **Excipients and active substances** | **Function** | **Table t A** | **Tablet B** | **Tablet C** | **Tablet D** | **Tablet E** |
|---|---|---|---|---|---|---|
| Enalapril maleate | active | 20.0 | 20.0 | 20.0 | 20.0 | 20.0 |
| Lercanidipine HCl | active | 10.0 | 10.0 | 10.0 | 10.0 | 10.0 |
| Hydroxypropyl cellulose | binder | 10.0 | 10.0 | 10.0 | 10.0 | / |
| Povidone | binder | / | / | / | / | 10.0 |
| Maleic acid | stabilizer | 5.0 | 1.0 | 5.0 | 5.0 | 5.0 |
| Sodium starch glycolate | disintegrant | 20.0 | 20.0 | 20.0 | 20.0 | 20.0 |
| Mannitol | filler | 220.0 | 220.0 | 220.0 | 220.0 | 220.0 |
| | | | | | | |
| Mannitol | filler | 91.0 | 95.0 | 91.0 | 87.0 | 87.0 |
| Sodium starch glycolate | disintegrant | 20.0 | 20.0 | 20.0 | 20.0 | 20.0 |
| Sodium stearyl fumarate | lubricant | 4.0 | 4.0 | 4.0 | 8.0 | 8.0 |
| **Total tablet core** | | **400.0** | **400.0** | **400.0** | **400.0** | **400.0** |
| | | | | | | |
| Coating agent | Coating agent | 10.0 | 10.0 | 10.0 | 10.0 | 10.0 |
| **Total tablet** | | **410.0** | **410.0** | **410.0** | **410.0** | **410.0** |

**Stability results of Tablets A, B, C, D and E prepared according to Example 1**

| | Stability conditions | Enalaprilate (III) (%) | DKP(IV) (%); EM | impurity with MH⁺ 331.1 Da (C₁₆H₁₅O₆N₂); resulting from degradation of lercanidipine (%) | Impurity with MH⁺ 418.2 Da (C₂₁H₂₈O₆N₃); resulting from degradation of lercanidipine (%) | Impurity with MH⁺ 598.3 Da (C₃₅H₄₀O₆N₃); resulting from degradation of lercanidipine (%) | impurity with MH⁺ 610.3 Da = LRCNA (C₃₆H₄₀O₆N₃); resulting from degradation of lercanidipine (%) |
|---|---|---|---|---|---|---|---|
| Example 1, Tablet A | t₀ | < 0.01 | 0.05 | < 0.01 | < 0.01 | < 0.01 | < 0.01 |
| | 50°C-closed - 7 days | 0.04 | 0.37 | < 0.01 | < 0.01 | < 0.01 | 0.07 |
| Example 1, Tablet B | t₀ | < 0.01 | 0.07 | < 0.01 | < 0.01 | < 0.01 | < 0.01 |
| | 50°C- closed - 7 days | 0.06 | 2.07 | < 0.01 | < 0.01 | < 0.01 | < 0.01 |
| Example 1, Tablet C | t₀ | < 0.01 | 0.07 | < 0.01 | < 0.01 | < 0.01 | < 0.01 |
| | 50°C- closed - 7 days | < 0.01 | 0.55 | < 0.01 | < 0.01 | < 0.01 | 0.11 |
| Example 1, Tablet D | t₀ | 0.11 | < 0.01 | < 0.01 | < 0.01 | < 0.01 | 0.04 |
| | 50°C- closed - 7 days | 0.12 | 0.33 | < 0.01 | < 0.01 | < 0.01 | 0.07 |
| Example 1, Tablet E | t₀ | 0.11 | 0.05 | < 0.01 | < 0.01 | < 0.01 | 0.03 |
| | 50°C- closed - 7 days | 0.13 | 0.31 | < 0.01 | < 0.01 | < 0.01 | 0.06 |

### Example 2

Povidone and maleic acid were dissolved in water. With the obtained granulation liquid, enalapril maleate, lercanidipine hydrochloride (amorphous), part of sodium starch glycolate and a part crystalline lactose were granulated. The obtained granules were dried. The dried granules were mixed with the second part of sodium starch glycolate, the second part of crystalline lactose, and magnesium stearate, and compressed into tablets. The obtained tablets were coated with the coating agent.

| **Excipients and active substances** | **Function** | **Tablet A** |
|---|---|---|
| Enalapril maleate | active | 20.0 |
| Lercanidipine HCl | active | 10.0 |
| Povidone | binder | 8.0 |
| Maleic acid | stabilizer | 10.0 |
| Sodium starch glycolate | disintegrant | 8.0 |
| Lactose crystalline | filler | 160.0 |
| | | |
| Lactose crystalline | filler | 155.0 |
| Sodium starch glycolate | disintegrant | 25.0 |
| Magnesium stearate | lubricant | 4.0 |
| **Total tablet core** | | **400.0** |
| | | |
| Coating agent | coating agent | 10.0 |
| **Total tablet** | | **410.0** |

**Stability results of Tablets A prepared according to Example 2**

| | Stability conditions | Enalaprilate (III) (%) | DKP(IV) (%); EM | Impurity with MH⁺ 331.1 Da (C₁₆H₁₅O₆N₂); resulting from degradation of lercanidipine (%) | Impurity with MH⁺ 418.2 Da (C₂₁H₂₈O₆N₃); resulting from degradation of lercanidipine (%) | Impurity with MH⁺ 598.3 Da (C₃₅H₄₀O₆N₃); resulting from degradation of lercanidipine (%) | impurity with MH⁺ 610.3 Da = LRCNA (C₃₆H₄₀O₆N₃); resulting from degradation of lercanidipine (%) |
|---|---|---|---|---|---|---|---|
| Example 2, Tablet A | t₀ | 0.02 | 0.07 | < 0.01 | 0.01 | 0.02 | 0.19 |
| | 50°C-closed - 7 days | 0.13 | 0.44 | < 0.01 | 0.01 | 0.01 | 0.28 |

### Example 3

Hydroxy propylcellulose and citric acid were dissolved in water. With the obtained granulation liquid, enalapril maleate, lercanidipine hydrochloride (amorphous), part of sodium starch glycolate and a part of mannitol were granulated in a fluid bed granulator. The obtained granules were dried. The dried granules were mixed with the second part of sodium starch glycolate, the second part of mannitol, and sodium stearyl fumarate, and compressed into tablets. The obtained tablets were coated with the coating agent.

| **Excipients and active substances** | **Function** | **Tablet A** | **Tablet B** |
|---|---|---|---|
| Enalapril maleate | active | 20.0 | 20.0 |
| Lercanidipine HCl | active | 10.0 | 10.0 |
| Hydroxypropyl cellulose | binder | 10.0 | 10.0 |
| Citric acid | stabilizer | 10.0 | 2.0 |
| Sodium starch glycolate | disintegrant | 15.0 | 15.0 |
| Mannitol | filler | 200.0 | 200.0 |
| | | | |
| Mannitol | filler | 106.0 | 114.0 |
| Sodium starch glycolate | disintegrant | 25.0 | 25.0 |
| Sodium stearyl fumarate | lubricant | 4.0 | 4.0 |
| **Total tablet core** | | **400.0** | **400.0** |
| | | | |
| Coating agent | Coating agent | 10.0 | 10.0 |
| **Total tablet** | | **410.0** | **410.0** |

**Stability results of Tablets A and B prepared according to Example 3**

| | Stability conditions | Enalaprilate (III) (%) | DKP(IV) (%); EM | impurity with MH⁺ 331.1 Da (C₁₆H₁₅O₆N₂); resulting from degradation of lercanidipine (%) | Impurity with MH⁺ 418.2 Da (C₂₁H₂₈O₆N₃); resulting from degradation of lercanidipine (%) | Impurity with MH⁺ 598.3 Da (C₃₅H₄₀O₆N₃); resulting from degradation of lercanidipine (%) | Impurity with MH⁺ 610.3 Da = LRCNA (C₃₆H₄₀D₆N₃); resulting from degradation of lercanidipine (%) |
|---|---|---|---|---|---|---|---|
| Example 3, Tablet A | t₀ | 0.04 | 0.10 | < 0.01 | < 0.01 | < 0.01 | 0.02 |
| | 50°C-closed - 7 days | 0.58 | 4.35 | < 0.01 | 0.03 | 0.03 | 0.23 |
| | 40°C-closed -1 month | 0.57 | 3.04 | < 0.01 | < 0.01 | < 0.01 | 0.16 |
| Example 3, Tablet B | to | 0.03 | 0.12 | < 0.01 | < 0.01 | < 0.01 | 0.04 |
| | 50°C-closed - 7 days | 0.15 | 4.27 | < 0.01 | 0.04 | 0.04 | 0.13 |
| | 40°C-closed -1 month | 0.14 | 3.04 | < 0.01 | 0.03 | 0.04 | 0.08 |

### Example 4

Binder (hydroxy propylcellulose or povidone) and maleic acid were dissolved in water. With the obtained granulation liquid, enalapril maleate, lercanidipine hydrochloride (polymorph V), part of sodium starch glycolate and crystalline lactose were granulated. The obtained granules were dried. The dried granules were mixed with the second part of sodium starch glycolate, mannitol, and sodium stearyl fumarate, and compressed into tablets. The obtained tablets were coated with the coating agent.

| **Excipients and active substances** | **Function** | **Tablet A** | **Tablet B** |
|---|---|---|---|
| Enalapril maleate | active | 20.0 | 20.0 |
| Lercanidipine HCl | active | 10.0 | 10.0 |
| Hydroxy propylcellulose | binder | 10.0 | / |
| Povidone | binder | / | 10.0 |
| Maleic acid | stabilizer | 5.0 | 5.0 |
| Sodium starch glycolate | disintegrant | 20.0 | 20.0 |
| Lactose crystalline | filler | 220.0 | 220.0 |
| | | | |
| Mannitol | filler | 87.0 | 87.0 |
| Sodium starch glycolate | disintegrant | 20.0 | 20.0 |
| Sodium stearyl fumarate | lubricant | 8.0 | 8.0 |
| **Total tablet core** | | **400.0** | **400.0** |
| | | | |
| Coating agent | coating agent | 10.0 | 10.0 |
| **Total tablet** | | **410.0** | **410.0** |

**Stability results of Tablets A and B prepared according to Example 4**

| | Stability conditions | Enala-prilate (III) (%) | DKP(IV) (%); EM | impurity with MH⁺ 331.1 Da (C₁₆H₁₅O₆N₂); resulting from degradation of lercanidipine (%) | Impurity with MH⁺ 418.2 Da (C₂₁H₂₈O₆N₃); resulting from degradation of lercanidipine (%) | impurity with MH⁺ 598.3 Da (C₃₅H₄₀O₆N₃); resulting from degradation of lercanidipine (%) | Impurity with MH⁺ 610.3 Da = LRCNA (C₃₆H₄₀C₆N₃); resulting from degradation of lercanidipine (%) |
|---|---|---|---|---|---|---|---|
| Example 4, Tablet A | t₀ | 0.11 | 0.03 | < 0.01 | < 0.01 | < 0.01 | 0.04 |
| | 50°C-closed - 7 days | 0.12 | 0.42 | < 0.01 | < 0.01 | < 0.01 | 0.06 |
| Example 4, Tablet B | to | 0.11 | < 0.01 | < 0.01 | < 0.01 | < 0.01 | 0.04 |
| | 50°C-closed - 7 days | 0.15 | 0.43 | < 0.01 | < 0.01 | < 0.01 | 0.05 |

### Example 5

Binder (hydroxy propylcellulose or povidone) and maleic acid were dissolved in water. With the obtained granulation liquid, enalapril maleate, lercanidipine hydrochloride (polymorph V), a part of sodium starch glycolate and a part of crystalline lactose were granulated. The obtained granules were dried. The dried granules were mixed with the second part of sodium starch glycolate, the second part of crystalline lactose and sodium stearyl fumarate, and compressed into tablets. The obtained tablets were coated with the coating agent.

| **Excipients and active substances** | **Function** | **Tablet A** | **Tablet B** |
|---|---|---|---|
| Enalapril maleate | active | 20.0 | 20.0 |
| Lercanidipine HCl | active | 10.0 | 10.0 |
| Hydroxy propylcellulose | binder | 10.0 | / |
| Povidone | binder | / | 10.0 |
| Maleic acid | stabilizer | 5.0 | 5.0 |
| Sodium starch glycolate | disintegrant | 20.0 | 20.0 |
| Lactose crystalline | filler | 220.0 | 220.0 |
| | | | |
| Lactose crystalline | filler | 87.0 | 87.0 |
| Sodium starch glycolate | disintegrant | 20.0 | 20.0 |
| Sodium stearyl fumarate | lubricant | 8.0 | 8.0 |
| **Total tablet core** | | **400.0** | **400.0** |
| | | | |
| Coating agent | coating agent | 10.0 | 10.0 |
| **Total tablet** | | **410.0** | **410.0** |

**Stability results of Tablets A and B prepared according to Example 5**

| | Stability conditions | Enalaprilate (III) (%) | DKP(IV) (%); EM | impurity with MH⁺ 331.1 Da (C₁₆H₁₅O₆N₂); resulting from degradation of lercanidipine (%) | Impurity with MH⁺ 418.2 Da (C₂₁H₂₆O₆N₃); resulting from degradation of lercanidipine (%) | Impurity with MH⁺ 598.3 Da (C₃₅H₄₀O₆N₃); resulting from degradation of lercanidipine (%) | Impurity with MH⁺ 610.3 Da = LRCNA (C₃₆H₄₀O₆N₃); resulting from degradation of lercanidipine (%) |
|---|---|---|---|---|---|---|---|
| Example 5, Tablet A | t₀ | 0.11 | 0.04 | < 0.01 | < 0.01 | < 0.01 | 0.03 |
| | 50°C-closed -7 days | 0.13 | 0.39 | < 0.01 | < 0.01 | < 0.01 | 0.07 |
| Example 5, Tablet B | to | 0.11 | < 0.01 | < 0.01 | < 0.01 | < 0.01 | 0.04 |
| | 50°C-closed - 7 days | 0.15 | 0.35 | < 0.01 | < 0.01 | < 0.01 | 0.07 |

### Example 6

Povidone and maleic acid were dissolved in water. With the obtained granulation liquid, enalapril maleate, a part of sodium starch glycolate and a part of crystalline lactose were granulated. The obtained granules were dried. The dried granules were mixed with lercanidipine hydrochloride (polymorph V), the second part of sodium starch glycolate, the second part of crystalline lactose and sodium stearyl fumarate, and compressed into tablets. The obtained tablets were coated with the coating agent.

| **Excipients and active substances** | **Function** | **Tablet A** |
|---|---|---|
| Enalapril maleate | active | 20.0 |
| Povidone | binder | 10.0 |
| Maleic acid | stabilizer | 5.0 |
| Sodium starch glycolate | disintegrant | 20.0 |
| Lactose crystalline | filler | 220.0 |
| | | |
| Lercanidipine HCl | active | 10.0 |
| Lactose crystalline | filler | 87.0 |
| Sodium starch glycolate | disintegrant | 20.0 |
| Sodium stearyl fumarate | lubricant | 8.0 |
| **Total tablet core** | | **400.0** |
| | | |
| Coating agent | coating agent | 10.0 |
| **Total tablet** | | **410.0** |

**Stability results of Tablet A prepared according to Example 6**

| | Stability conditions | Enalaprilate (III) (%) | DKP(IV) (%); EM | Impurity with MH⁺ 331.1 Da (C₁₆H₁₅O₆N₂); resulting from degradation of lercanidipine (%) | Impurity with MH⁺ 418.2 Da (C₂₁H₂₈O₆N₃); resulting from degradation of lercanidipine (%) | Impurity with MH⁺ 598.3 Da (C₃₅H₄₀O₆N₃); resulting from degradation of lercanidipine (%) | Impurity with MH⁺ 610.3 Da = LRCNA (C₃₆H₄₀O₆N₃); resulting from degradation of lercanidipine (%) |
|---|---|---|---|---|---|---|---|
| Example 6, Tablet A | t₀ | 0.12 | 0.04 | < 0.01 | < 0.01 | < 0.01 | 0.02 |
| | 50°C-closed - 7 days | 0.15 | 0.33 | < 0.01 | < 0.01 | < 0.01 | 0.04 |

## Claims

1. Combined pharmaceutical solid oral dosage form comprising lercanidipine or a pharmaceutically acceptable salt thereof as the active substance, and enalapril or a pharmaceutically acceptable salt thereof as the active substance, and comprising an organic acid, wherein the organic acid is maleic and/or citric acid.

2. Combined homogenous pharmaceutical oral dosage form according to claim 1 comprising lercanidipine or a pharmaceutically acceptable salt thereof as the active substance, and enalapril or a pharmaceutically acceptable salt thereof as the active substance, and comprising an organic acid to stabilize both enalapril and lercanidipine, and their pharmaceutically acceptable salts.

3. Combined pharmaceutical oral dosage form according to claim 1 or 2 comprising lercanidipine hydrochloride and enalapril maleate.

4. Combined pharmaceutical oral dosage form according to any one of claims 1 to 3, wherein at least one of the active substances lercanidipine and enalapril or their pharmaceutically acceptable salts is incorporated into a granulate or wherein both lercanidipine and enalapril or their pharmaceutically acceptable salts are together and/or simultaneously incorporated into a granulate.

5. Combined pharmaceutical oral dosage form according to any one of claims 1 to 4 comprising amorphous lercanidipine hydrochloride either in purely amorphous form or comprising 1 wt% to 5 wt% of a crystalline form, form V, or form I lercanidipine hydrochloride.

6. Combined pharmaceutical oral dosage form according to any one of claims 1 to 5 **characterized in that** it is in the form of a granulate, granules, grains, beads or pellets, which are mixed and filled into capsules or sachets or are compressed to tablets.

7. Combined pharmaceutical oral dosage form according to any one of claims 1 to 6 comprising mannitol and/or lactose as fillers.

8. Combined pharmaceutical oral dosage form according to any one of claims 1 to 7 comprising povidone or hydroxypropyl cellulose as binder.

9. Combined pharmaceutical oral dosage form according to any one of claims 1 to 8 comprising 0.05 to 15.0, preferably 0.05 to 7.0, more preferably 0.1 to 5.0, even more preferably 0.1 to 3.0 and most preferably 0.2 to 2.5% by weight of the organic acid.

10. Combined pharmaceutical oral dosage form according to any of claims 1 to 9 comprising lercanidipine or a pharmaceutically acceptable salt thereof, preferably lercanidipine hydrochloride, wherein the d50 particle size is less than 50 micrometers, preferably less than 30 micrometers and most preferably less than 20 micrometers.

11. A method for preparing a combined pharmaceutical oral dosage form according to any one of claims 1 to 10 comprising maleic and/or citric acid, which method comprises the steps of:
i.) dissolving the pharmaceutically active substances in water, a suitable organic solvent, or molten binder, or a mixture thereof, optionally with some of the pharmaceutically inactive excipients used, and granulating the remaining pharmaceutically inactive excipients therewith, or
ii.) dissolving a part of the pharmaceutically active substances in water, a suitable organic solvent, or molten binder, or a mixture thereof, optionally with some of the pharmaceutically inactive excipients used, and granulating the remaining pharmaceutically active and inactive excipients therewith, or
iii.) dissolving some of the pharmaceutically inactive excipients in water, a suitable organic solvent, or molten binder, or a mixture thereof, and granulating the pharmaceutically active substance and the pharmaceutically inactive excipients therewith.

12. Method for preparing a combined pharmaceutical oral dosage form according to claim 11, which comprises the steps of:
i.) dissolving or suspending the organic acid in a liquid to prepare a granulation liquid or suspension, wherein the organic acid is maleic and/or citric acid,
ii.) granulating therewith a mixture of both active ingredients lercanidipine and enalapril or their pharmaceutically acceptable salts, optionally together with other pharmaceutically acceptable excipients or part of pharmaceutically acceptable excipients used,
iii.) drying the granulate at a temperature of 20 to 90°C for 1 to 80 minutes,
iv.) admixing other pharmaceutically acceptable excipients or part of pharmaceutically acceptable excipients used to the granulate prepared under iii.),
v.) compressing the mixture obtained under iv.) into a tablet at a pressure of 1 to 40 kN, and
vi.) optionally coating the tablet obtained under v.).

13. A method for preparing a combined pharmaceutical oral dosage form according to any one of claims 1 to 10 , which comprises the steps of:
i.) dissolving or suspending the organic acid and optionally other pharmaceutically acceptable excipients in a liquid to prepare a granulation liquid or suspension, wherein the organic acid is maleic and/or citric acid,
ii.) granulating therewith the active substance enalapril or its pharmaceutically acceptable salt optionally together with other pharmaceutically acceptable excipients or part of pharmaceutically acceptable excipients used,
iii.) drying the granulate at a temperature of 20 to 90 °C for 1 to 80 minutes,
iv.) mixing the dried granulate obtained under iii.) with the active substance lercanidipine or its pharmaceutically acceptable salt and other pharmaceutically acceptable excipients or part of pharmaceutically acceptable excipients used,
v.) compressing the mixture obtained under iv.) into a tablet at a pressure of 1 to 40 kN,
vi.) optionally coating the tablet obtained under v.).

14. Method according to any one of claims 11 to 13, wherein the combined pharmaceutical oral dosage form is prepared by fluid bed granulation or by aqueous granulation.

## Patentansprüche

1. Pharmazeutische feste orale Kombinationsdarreichungsform, die Lercanidipin oder ein pharmazeutisch annehmbares Salz davon als Wirkstoff und Enalapril oder ein pharmazeutisch annehmbares Salz davon als Wirkstoff und eine organische Säure enthält, wobei die organische Säure Maleinsäure und/oder Zitronensäure ist.

2. Homogene pharmazeutische orale Kombinationsdarreichungsform nach Anspruch 1, die Lercanidipin oder ein pharmazeutisch annehmbares Salz davon als Wirkstoff und Enalapril oder ein pharmazeutisch annehmbares Salz davon als Wirkstoff und eine organische Säure enthält, um sowohl Enalapril als auch Lercanidipin sowie ihre pharmazeutisch annehmbaren Salze zu stabilisieren.

3. Pharmazeutische orale Kombinationsdarreichungsform nach Anspruch 1 oder 2, die Lercanidipinhydrochlorid und Enalaprilmaleat enthält.

4. Pharmazeutische orale Kombinationsdarreichungsform nach einem der Ansprüche 1 bis 3, wobei mindestens einer der Wirkstoffe Lercanidipin und Enalapril oder ihre pharmazeutisch annehmbaren Salze in ein Granulat eingebracht ist oder wobei sowohl Lercanidipin als auch Enalapril oder ihre pharmazeutisch annehmbaren Salze zusammen und/oder gleichzeitig in ein Granulat eingebracht sind.

5. Pharmazeutische orale Kombinationsdarreichungsform nach einem der Ansprüche 1 bis 4, die amorphes Lercanidipinhydrochlorid entweder in rein amorpher Form oder mit 1 Gew.-% bis 5 Gew.-% einer kristallinen Form, Form V oder Form I Lercanidipinhydrochlorid enthält.

6. Pharmazeutische orale Kombinationsdarreichungsform nach einem der Ansprüche 1 bis 5, **dadurch gekennzeichnet, dass** sie in Form eines Granulats, Granalien, Körnern, Kügelchen oder Pellets vorliegt, die gemischt und in Kapseln oder Beutel gefüllt oder zu Tabletten gepresst werden.

7. Pharmazeutische orale Kombinationsdarreichungsform nach einem der Ansprüche 1 bis 6, die Mannit und/oder Lactose als Füllstoff enthält.

8. Pharmazeutische orale Kombinationsdarreichungsform nach einem der Ansprüche 1 bis 7, die Povidon oder Hydroxypropylcellulose als Bindemittel enthält.

9. Pharmazeutische orale Kombinationsdarreichungsform nach einem der Ansprüche 1 bis 8, die 0,05 bis 15,0, bevorzugt 0,05 bis 7,0, bevorzugter 0,1 bis 5,0, noch bevorzugter 0,1 bis 3,0 und am bevorzugtesten 0,2 bis 2,5 Gew.-% der organischen Säure enthält.

10. Pharmazeutische orale Kombinationsdarreichungsform nach einem der Ansprüche 1 bis 9, die Lercanidipin oder ein pharmazeutisch annehmbares Salz davon enthält, vorzugsweise Lercanidipinhydrochlorid, wobei die d50-Teilchengröße weniger als 50 Mikrometer, bevorzugt weniger als 30 Mikrometer und am bevorzugtesten weniger als 20 Mikrometer beträgt.

11. Verfahren zur Herstellung einer pharmazeutischen oralen Kombinationsdarreichungsform gemäß einem der Ansprüche 1 bis 10, die Maleinsäure und/oder Zitronensäure enthält, bei dem
i.) die pharmazeutischen Wirkstoffe gegebenenfalls mit einigen der verwendeten pharmazeutisch inaktiven Hilfsstoffen in Wasser, einem geeigneten organischen Lösemittel, einem geschmolzenen Bindemittel oder einer Mischung davon gelöst werden und die übrigen pharmazeutisch inaktiven Hilfsstoffe damit granuliert werden oder
ii.) ein Teil der pharmazeutischen Wirkstoffe gegebenenfalls mit einigen der verwendeten pharmazeutisch inaktiven Hilfsstoffe in Wasser, einem geeigneten organischen Lösemittel, einem geschmolzenen Bindemittel oder einer Mischung davon gelöst wird und die übrigen pharmazeutisch aktiven und inaktiven Stoffe damit granuliert werden oder
iii.) einige der pharmazeutisch inaktiven Hilfsstoffe in Wasser, einem geeigneten organischen Lösemittel, einem geschmolzenen Bindemittel oder einer Mischung davon gelöst werden und die pharmazeutischen Wirkstoffe und die pharmazeutisch inaktiven Hilfsstoffe damit granuliert werden.

12. Verfahren zur Herstellung einer pharmazeutischen oralen Kombinationsdarreichungsform nach Anspruch 11, bei dem
i.) die organische Säure in einer Flüssigkeit gelöst oder suspendiert wird, um eine Granulationsflüssigkeit oder -suspension herzustellen, wobei die organische Säure Maleinsäure und/oder Zitronensäure ist,
ii.) eine Mischung aus beiden Wirkstoffen Lercanidipin und Enalapril oder ihren pharmazeutisch annehmbaren Salzen gegebenenfalls zusammen mit anderen pharmazeutisch annehmbaren Hilfsstoffen oder Teilen von verwendeten pharmazeutisch annehmbaren Hilfsstoffen damit granuliert wird
iii.) das Granulat bei einer Temperatur von 20°C bis 90°C für 1 bis 80 Minuten getrocknet wird,
iv.) andere pharmazeutisch annehmbare Hilfsstoffe oder Teile von verwendeten pharmazeutisch annehmbaren Hilfsstoffen zu dem in iii.) hergestellten Granulat gemischt werden,
v.) die in iv.) erhaltene Mischung bei einem Druck von 1 bis 40 kN zu einer Tablette gepresst wird und
vi.) die in v.) erhaltene Tablette gegebenenfalls beschichtet wird.

13. Verfahren zur Herstellung einer pharmazeutischen oralen Kombinationsdarreichungsform gemäß einem der Ansprüche 1 bis 10, bei dem
i.) die organische Säure und gegebenenfalls andere pharmazeutisch annehmbare Hilfsstoffe in einer Flüssigkeit gelöst oder suspendiert werden, um eine Granulationsflüssigkeit oder -suspension herzustellen, wobei die organische Säure Maleinsäure und/oder Zitronensäure ist,
ii.) der Wirkstoff Enalapril oder dessen pharmazeutisch annehmbares Salz gegebenenfalls zusammen mit anderen pharmazeutisch annehmbaren Hilfsstoffen oder Teilen von verwendeten pharmazeutisch annehmbaren Hilfsstoffen damit granuliert wird,
iii.) das Granulat bei einer Temperatur von 20°C bis 90°C für 1 bis 80 Minuten getrocknet wird,
iv.) das in iii.) erhaltene, getrocknete Granulat mit dem Wirkstoff Lercanidipin oder dessen pharmazeutisch annehmbaren Salz und anderen pharmazeutisch annehmbaren Hilfsstoffen oder Teilen von verwendeten pharmazeutisch annehmbaren Hilfsstoffen gemischt wird,
v.) die in iv.) erhaltene Mischung bei einem Druck von 1 bis 40 kN zu einer Tablette gepresst wird und
vi.) die in v.) erhaltene Tablette gegebenenfalls beschichtet wird.

14. Verfahren nach einem der Ansprüche 11 bis 13, wobei die pharmazeutische orale Kombinationsdarreichungsform mittels Wirbelschichtgranulation oder wässriger Granulation hergestellt wird.

## Revendications

1. Forme posologique pharmaceutique solide combinée pour voie orale comprenant, en tant que substance active, de la lercanidipine ou de l'un de ses sels pharmacologiquement admissibles, ainsi que, en tant que substance active, de l'énalapril ou de l'un de ses sels pharmacologiquement admissibles, et comprenant un acide organique, lequel acide organique est de l'acide maléique et/ou de l'acide citrique.

2. Forme posologique pharmaceutique homogène combinée pour voie orale, conforme à la revendication 1, comprenant, en tant que substance active, de la lercanidipine ou de l'un de ses sels pharmacologiquement admissibles, ainsi que, en tant que substance active, de l'énalapril ou de l'un de ses sels pharmacologiquement admissibles, et comprenant un acide organique afin de stabiliser à la fois l'énalapril et la lercanidipine ou leurs sels pharmacologiquement admissibles.

3. Forme posologique pharmaceutique combinée pour voie orale, conforme à la revendication 1 ou 2, comprenant du chlorhydrate de lercanidipine et du maléate d'énalapril.

4. Forme posologique pharmaceutique combinée pour voie orale, conforme à l'une des revendications 1 à 3, dans laquelle au moins l'une des substances actives, lercanidipine et énalapril ou leurs sels pharmacologiquement admissibles, est incorporée dans un granulat, ou bien les deux substances lercanidipine et énalapril ou leurs sels pharmacologiquement admissibles sont conjointement et/ou simultanément incorporées dans un granulat.

5. Forme posologique pharmaceutique combinée pour voie orale, conforme à l'une des revendications 1 à 4, comprenant du chlorhydrate de lercanidipine amorphe, soit sous forme amorphe pure, soit sous une forme comprenant de 1 % en poids à 5 % en poids d'une forme cristalline, la forme V ou la forme I, du chlorhydrate de lercanidipine.

6. Forme posologique pharmaceutique combinée pour voie orale, conforme à l'une des revendications 1 à 5, **caractérisée en ce qu'**elle se présente sous forme de granulat, de granules, de grains, de perles ou de pilules, qu'on mélange et dont on remplit des capsules ou des sachets ou dont on fait des comprimés par compression.

7. Forme posologique pharmaceutique combinée pour voie orale, conforme à l'une des revendications 1 à 6, comprenant du mannitol et/ou du lactose, en tant que charge(s).

8. Forme posologique pharmaceutique combinée pour voie orale, conforme à l'une des revendications 1 à 7, comprenant de la povidone ou de l'hydroxypropyl-cellulose en tant que liant.

9. Forme posologique pharmaceutique combinée pour voie orale, conforme à l'une des revendications 1 à 8, comprenant de 0,05 à 15,0, de préférence de 0,05 à 7,0, mieux encore de 0,1 à 5,0, encore mieux de 0,1 à 3,0, et surtout de 0,2 à 2,5 % en poids d'acide organique.

10. Forme posologique pharmaceutique combinée pour voie orale, conforme à l'une des revendications 1 à 9, comprenant de la lercanidipine ou de l'un de ses sels pharmacologiquement admissibles, de préférence du chlorhydrate de lercanidipine, dont la taille de particules d₅₀ vaut moins de 50 micromètres, de préférence moins de 30 micromètres et surtout moins de 20 micromètres.

11. Procédé de préparation d'une forme posologique pharmaceutique combinée pour voie orale conforme à l'une des revendications 1 à 10, comprenant de l'acide maléique et/ou de l'acide citrique, lequel procédé comporte les étapes suivantes :
i) dissoudre les substances pharmaceutiquement actives dans de l'eau, un solvant organique approprié ou un liant fondu, ou un mélange de telles substances, en option avec certains des excipients pharmaceutiquement inactifs utilisés, et granuler avec cela le reste des excipients pharmaceutiquement inactifs ;
ii) ou dissoudre une partie des substances pharmaceutiquement actives dans de l'eau, un solvant organique approprié ou un liant fondu, ou un mélange de telles substances, en option avec certains des excipients pharmaceutiquement inactifs utilisés, et granuler avec cela le reste des substances pharmaceutiquement actives et des excipients inactifs,
iii) ou dissoudre certains des excipients pharmaceutiquement inactifs dans de l'eau, un solvant organique approprié ou un liant fondu, ou un mélange de telles substances, et granuler avec cela les substances pharmaceutiquement actives et les excipients pharmaceutiquement inactifs.

12. Procédé de préparation d'une forme posologique pharmaceutique combinée pour voie orale, conforme à la revendication 11, qui comporte les étapes suivantes :
i) dissoudre l'acide organique ou le mettre en suspension dans un liquide, pour préparer un liquide ou une suspension pour granulation, étant entendu que l'acide organique est de l'acide maléique et/ou de l'acide citrique ;
ii) granuler avec cela un mélange des deux ingrédients actifs, lercanidipine et énalapril ou leurs sels pharmacologiquement admissibles, en option conjointement avec d'autres excipients pharmacologiquement admissibles ou une partie des excipients pharmacologiquement admissibles utilisés ;
iii) faire sécher le granulat, à une température de 20 à 90 °C et pendant 1 à 80 minutes ;
iv) ajouter et mélanger au granulat préparé dans l'étape (iii) d'autres excipients pharmacologiquement admissibles ou une partie des excipients pharmacologiquement admissibles utilisés ;
v) comprimer le mélange obtenu à l'issue de l'étape (iv), sous une pression de 1 à 40 kN, pour en faire un comprimé ;
vi) et en option, enrober le comprimé obtenu à l'issue de l'étape (v).

13. Procédé de préparation d'une forme posologique pharmaceutique combinée pour voie orale conforme à l'une des revendications 1 à 10, lequel procédé comporte les étapes suivantes :
i) dissoudre ou mettre en suspension dans un liquide l'acide organique, et en option, d'autres excipients pharmacologiquement admissibles, pour préparer un liquide ou une suspension pour granulation, étant entendu que l'acide organique est de l'acide maléique et/ou de l'acide citrique ;
ii) granuler avec cela la substance active énalapril ou l'un de ses sels pharmacologiquement admissibles, en option conjointement avec d'autres excipients pharmacologiquement admissibles ou une partie des excipients pharmacologiquement admissibles utilisés ;
iii) faire sécher le granulat, à une température de 20 à 90 °C et pendant 1 à 80 minutes ;
iv) mélanger le granulat séché obtenu à l'issue de l'étape (iii) avec la substance active lercanidipine ou l'un de ses sels pharmacologiquement admissibles, et d'autres excipients pharmacologiquement admissibles ou une partie des excipients pharmacologiquement admissibles utilisés ;
v) comprimer le mélange obtenu à l'issue de l'étape (iv), sous une pression de 1 à 40 kN, pour en faire un comprimé ;
vi) et en option, enrober le comprimé obtenu à l'issue de l'étape (v).

14. Procédé conforme à l'une des revendications 11 à 13, dans lequel la forme posologique pharmaceutique combinée pour voie orale est préparée par granulation en lit fluidisé ou par granulation par voie humide aqueuse.
